# EUROPÄISCHE PATENTANMELDUNG

(11) **EP 3 263 557 A1**
(43) Veröffentlichungstag der Anmeldung: **03.01.2018**
(21) Anmeldenummer: 16177260.3
(22) Anmeldetag: 30.06.2016
(51) Int. Cl.: C07D 225/02, B01J 38/64, B01J 38/66, B01J 23/30, C07C 45/58, C07C 249/08, C07D 301/12

(54) **VERFAHREN ZUR REAKTIVIERUNG EINES HOMOGENEN OXIDATIONSKATALYSATORS**

(71) Anmelder: Evonik Degussa GmbH, 45128 Essen (DE)
(72) Erfinder: MEIER, Ralf, 44265 Dortmund (DE); BAJUS, Stephanie, 63450 Hanau (DE); DÖRING, Jens, 44141 Dortmund (DE)

(57) **Zusammenfassung**

Die vorliegende Erfindung betrifft ein Verfahren zur Reaktivierung homogener Katalysatorsysteme aus organischen Reaktionsmischungen. Die Katalysatorsysteme eignen sich für die Oxidation organischer Verbindungen wie beispielsweise Cyclododecen. Die Reaktivierung erfolgt mittels wässriger Base.

## Beschreibung

Die vorliegende Erfindung betrifft ein Verfahren zur Reaktivierung eines homogenen Oxidationskatalysators. Des Weiteren betrifft die Erfindung ein Verfahren zur Oxidation organischer Verbindungen. Darüber hinaus betrifft die Erfindung ein Verfahren zur Synthese von Laurinlactam sowie Polyamid 12.

Cyclododecanon (CDON) wird für die Synthese von Laurinlactam eingesetzt. Das Lactam wiederum eignet sich für die Herstellung von Polyamid 12. Die Herstellung von CDON kann ausgehend von Cyclododecatrien (CDT) erfolgen. Zunächst kann eine Selektivhydrierung von Cyclododecatrien (CDT) zu Cyclododecen (CDEN) vorgenommen werden. Anschließend folgen eine Epoxidierung von CDEN zu Mono-Epoxycyclododecan (CDANepoxid) und die Umlagerung von CDANepoxid zu Cyclododecanon (CDON).

Die Epoxidierung des CDEN kann beispielsweise in einem Zweiphasensystem in Gegenwart eines homogenen Katalysatorsystems erfolgen. Gemäß EP-A-2946831 (US 2015/0328619) wird das epoxidierte Produkt vom verbleibenden Reaktionsgemisch an einer Membran abgetrennt. Das Katalysatorsystem verbleibt im Retentant und kann dem Reaktionsgemisch im Kreislauf als kontinuierliches Verfahren wieder zugeführt werden.

Hierbei ist allerdings beobachtet worden, dass der Umsatz bei konstanter Katalysatorkonzentration im Laufe der Zeit abnimmt. Ein abnehmender Umsatz lässt sich durch die Zufuhr frischen Katalysators kompensieren. Hierbei wird ein Teil des eingesetzten Katalysators durch neuen Katalysator ersetzt.

Das Hinzuführen neuen Katalysators hat allerdings den Nachteil, dass der Verbrauch an Katalysator ansteigt. Neben steigenden Kosten führt dies vor allem zu einer erhöhten Belastung der Umwelt.

Weiterhin ist beobachtet worden, dass sich der Katalysator insbesondere bei kontinuierlichen Verfahren immer weiter aufkonzentriert. Als Folge wandelt sich der Katalysator in weniger reaktive Spezies um. Weiterhin fällt der Katalysator an vielen Stellen der Apparatur oder Anlage aus. Der Feststoff stört die Reaktionsführung und verstopft eingesetzte Membranfilter.

Es bestand nunmehr die Aufgabe, ein verbessertes Verfahren zur Oxidation organischer Verbindungen zur Verfügung zu stellen. Hierbei sollte der Umsatz der eingesetzten organischen Verbindung möglichst konstant gehalten werden. Weiterhin sollte insbesondere die Menge an neuem Katalysator verringert werden, um die Umweltbelastung nachhaltig zu vermeiden. Darüber hinaus sollte die Bildung von ausgefallenem Katalysator vermieden werden.

Demgemäß wurde ein neues Verfahren gemäß Anspruch 1 gefunden, das eine Reaktivierung des eingesetzten Katalysators erlaubt. Hierdurch kann die Menge an frischen Katalysator verringert werden oder vollständig auf frischen Katalysator verzichtet werden. In dem Verfahren wird eine organische Verbindung in einem Reaktionsgemisch unter Erhalt eines oxidierten Produkts oxidiert. Das Reaktionsgemisch umfasst ein homogenes Katalysatorsystem, welches die Oxidation der organischen Verbindung katalysiert, sowie mindestens ein Peroxid. Das Katalysatorsystem umfasst mindestens ein Metall in seiner höchsten Oxidationsstufe, wobei das Metall ausgewählt ist aus mindestens einem Metall der Gruppen IVb, Vb und Vlb des Periodensystems. Hierzu zählen insbesondere Titan (höchste Oxidationsstufe: 4), Zirconium (4), Hafnium (4), Vanadium (5), Niob (5), Tantal (5), Chrom (6), Molybdän (6) und Wolfram (6). Die Reaktivierung des Katalysatorsystems erfolgt durch Zugabe mindestens einer wässrigen Base auf einen pH-Wert ≥ 4, vorzugsweise ≥ 4,5, bevorzugt ≥ 7und besonders bevorzugt ≥ 11.

Durch das erfindungsgemäße Verfahren ist es möglich, das Ausfallen des Katalysatorsystems zu vermeiden. Das Aufkonzentrieren des Katalysatorsystems kann unterbunden werden, wobei im Vergleich zum Stand der Technik ein gleichhoher Umsatz realisiert werden kann.

Weitere Ausgestaltungen der Erfindung finden sich in den weiteren Ansprüchen.

Oxidationen im Sinne der Erfindung erfasst Epoxidierungen. Epoxidierungen sind bevorzugte Oxidationen.

Unter dem Begriff organische Verbindungen werden im Rahmen dieser Erfindung Verbindungen verstanden, die mindestens 2 Kohlestoffatome enthalten, linear oder cyclisch sind und eine oder mehrere Doppelbindungen enthalten können. Vorzugsweise werden ungesättigte organische Verbindungen eingesetzt. Bevorzugt sind Verbindungen mit sechs bis zwölf Kohlenstoffatomen, wobei diese besonders bevorzugt cyclisch sind. Ganz besonders bevorzugt sind cyclische, ungesättigte C12-Verbindungen, insbesondere CDEN. Aus CDEN entsteht durch die Reaktion CDANepoxid.

Geeignete Peroxide sind dem Fachmann bekannt. Hierzu zählen 3-Chlorperoxybenzoesäure, Peroxybenzoesäure, Peroxyessigsäure, Peroxybenzimidsäure, tert-Butylhydroperoxid, Dimethyldioxiran, Kaliumhydrogenperoxomonosulfat und Wasserstoffperoxid, wobei Wasserstoffperoxid bevorzugt ist.

Geeignete Katalysatorsysteme enthalten Übergangsmetalle, insbesondere Wolfram, Molybdän oder Vanadium, wie sie beispielsweise in WO 00/44704 A1 (AU 2299400 A) oder DE 3027349 A1 (GB 2055821 B) beschrieben sind. Das Katalysatorsystem kann die katalytisch wirkende Substanz selbst sein oder eine Mischung mit Wasser oder organischen Lösemitteln. Die organische Verbindung kann als organisches Lösemittel fungieren.

Das Katalysatorsystem umfasst vorzugsweise ein Derivat eines Metalls, das ausgewählt ist aus Wolfram, Molybdän und Vanadium. Als Derivat kommt beispielweise ein Oxid, ein gemischtes Oxid, eine sauerstoffhaltige Säure, ein Salz einer sauerstoffhaltigen Säure, ein Carbonylderivat, ein Sulfid, ein Chlorid, ein Oxichlorid oder ein Alkanoat der Elemente Wolfram, Molybdän und/oder Vanadium in Betracht.

Geeignete Derivate sind beispielsweise die Metallcarbonyle W(CO)₆ oder Mo(CO)₆, die Oxide MoO₂, MoO₅, Mo₂O₃, MoO₃, WO₂, W₂O₅, WO₃, VO₂, V₂O₃, oder V₂O₅, die Sulfide WS₂ oder WS₃. Weitere Beispiele sind die Sauerstoffsäuren H₂WO₄ und H₂MoO₄ bzw. deren Alkali- oder Erdalkalisalze. Beispielsweise umfassen bekannte Epoxidierungskatalysatoren Wolframat oder Molybdat, insbesondere Natriumwolframat, Na₂WO₄, oder Natriummolybdat, Na₂MoO₄. Weiterhin kann das Metall des Katalysatorsystems als Polyoxometallat vorliegen.

Diese Verbindungen werden meist in situ in die katalytisch aktive Verbindung umgewandelt. Dies erfolgt bevorzugt durch Umsetzung mit einem Derivat von Phosphor, Silicium und/oder Arsen. Besonders eignet sich hierfür ein Oxid, eine Sauerstoffsäure, ein Salz einer Sauerstoffsäure, ein Sulfid, ein Chlorid, ein Oxychlorid oder ein Fluorid von Phosphor, Silicium und/oder Arsen.

Bevorzugt umfasst das Katalysatorsystem deshalb eine katalytisch aktive Verbindung, die durch Umsetzung eines Derivats von Wolfram, Molybdän oder Vanadium mit einem Derivat von Phosphor oder Arsen erhalten wird. In einer besonders bevorzugten Ausführungsform wird die katalytisch aktive Übergangsmetallverbindung in situ durch Umsetzung von Natriumwolframat mit Phosphorsäure gebildet.

In einer besonders bevorzugten Ausführungsform der vorliegenden Erfindung umfasst das Katalysatorsystem demnach Phosphorsäure und ein Derivat eines Metalls, das ausgewählt ist aus Wolfram, Molybdän und Vanadium, welches in Verbindung mit einem Phasentransferreagenz eingesetzt wird (das Phasentransferreagenz bildet somit nicht einen Bestandteil des Katalysatorsystems).

Das Reaktionsgemisch umfasst vorzugsweise zwei nicht oder schwer mischbare flüssige Phasen. Eine der Phasen enthält im Wesentlichen Wasser (wässrige Phase). Daneben kann diese Phase das Peroxid, das Katalysatorsystem, Phasentransferreagenz und Spuren der organischen Verbindung sowie ihrer Folgeprodukte enthalten. Die andere Phase (organische Phase) enthält typischerweise im Wesentlichen das Reaktionsprodukt der Oxidation. Daneben kann diese Phase die organische Verbindung sowie das Katalysatorsystem und das Phasentransferreagenz enthalten. Insofern ist das Reaktionsgemisch vorzugsweise ein Mehrphasensystem umfassend eine wässrige und eine organische Phase, wobei weiterhin ein Phasentransferreagenz enthalten ist. Die beschriebenen Katalysatoren werden häufig in Verbindung mit einem Phasentransferreagenz eingesetzt, mit dessen Hilfe der an sich wasserlösliche Katalysator in die organische Phase überführt werden kann.

Als Phasentransferreagenz eignen sich hierbei insbesondere ternäre und quartäre Ammoniumverbindungen sowie Esterquats, wie sie beispielsweise in EP-A-2946831 beschrieben sind. Bevorzugt sind Amine oder Ammoniumsalze, wobei quartäre Ammoniumsalze besonders bevorzugt sind. Ein Beispiel für ein geeignetes Phasentransferreagenz ist das unter dem Namen Alamin erhältliche Trioctylamin. Die Octylreste können zumindest teilweise durch Decylreste ersetzt worden sein. Beispiele für quatäre Ammoniumverbindungen sind Trialkylammoniummethyl-salze, wobei die Alkylketten aus jeweils sechs bis zwölf C-Atomen bestehen. Das Trialkylammoniummethyl-sulfat ist ein ganz besonders bevorzugtes Phasentransferreagenz dieser Erfindung.

Die Reaktionsgeschwindigkeit der Oxidation ist, soweit eine wässrige Phase umfasst ist, abhängig vom pH-Wert. Insofern ist es bevorzugt, die Oxidation bei einem pH ≤ 4, vorzugsweise in einem Bereich von 1,5 bis 4, stattfinden zu lassen. Die Einstellung des pH-Werts erfolgt vorzugsweise mit mindestens einer anorganischen Säure, die bei 25 °C einen pKₛ-Wert (bzw. einen pKₛ₁-Wert) von 2,5 oder weniger aufweist. Geeignete Säuren werden ausgewählt aus Phosphorsäure, Salpetersäure, Schwefelsäure, Salzsäure, Perchlorsäure und ihren Mischungen, wobei bevorzugt Phosphorsäure, Schwefelsäure oder ihre Mischungen und besonders bevorzugt Schwefelsäure eingesetzt wird.

Die Oxidation kann in einem organischen Lösemittel durchgeführt werden. Alternativ kann die Oxidation ohne Lösemittel erfolgen, da die organische Verbindung selbst als Lösemittel fungieren kann.

Die Reaktivierung des Katalysatorsystems erfolgt mittels wässriger Base. Bevorzugte Basen sind aus Ammoniak, Alkalihydroxiden oder Mischungen daraus ausgewählt. Natrium und Kalium sind bevorzugte Alkalimetalle, wobei Natrium besonders bevorzugt ist. Geeignete Basen sind Natronlauge oder Kalilauge. Die Lösung der Base wird derart zugegeben, dass der gewünschte pH-Wert eingestellt ist. Durch eine chemische Reaktion wird der Katalysator reaktiviert.

Bevor die Reaktivierung vorgenommen wird, kann die oxidierte organische Verbindung (Produkt) und gegebenenfalls das Lösemittel vom Katalysatorsystem abgetrennt werden. Dadurch wird das Katalysatorsystem aufkonzentriert. Dies kann beispielsweise mittels Destillation, Extraktion, Kristallisation oder Membranfiltration erfolgen, wobei die Membranfiltration bevorzugt ist. Der Fachmann ist mit derartigen Abtrennungsmethoden vertraut. Hinsichtlich der Membranfiltration wird insbesondere auf EP-A-2946831 verwiesen. Die Membranfiltration weist den Vorteil auf, dass die Reaktion und Reaktivierung im Kreislauf als kontinuierliches Verfahren durchgeführt werden kann. Das aufkonzentrierte Katalysatorsystem (Retentat) wird im Anschluss mit der wässrigen Base vermischt.

Nach der Reaktivierung kann der Katalysator dem Reaktionsgemisch wieder zugeführt werden (kontinuierliche Verfahrensweise). Alternativ kann der Katalysator zwischengelagert und für spätere Reaktionen eingesetzt werden (Batch-Verfahrensweise), wobei eine kontinuierliche Verfahrensweise bevorzugt ist. Die Zuführung kann erfolgen, indem diejenige Phase zurückgeführt wird, in der sich der Katalysator befindet. Die Verteilung des metallischen Katalystors in den jeweiligen Phasen kann durch den pH-Wert gesteuert werden.

Nach der Reaktivierung und vor der Zuführung zu einem Reaktionsgemisch erfolgt vorzugsweise die Einstellung des pH-Werts in der sich ergebenden wässrigen Phase. Diese Maßnahme stellt sicher, dass keine Zersetzung des Peroxids, insbesondere des Wasserstoffperoxids, im Reaktionsgemisch erfolgt. Der pH-Wert sollte vorteilhafterweise in einem Bereich von < 7, vorzugsweise 1,5 - 4 eingestellt werden. Die Einstellung des pH-Werts wird vorzugsweise mit einer anorganischen Säure vorgenommen, die einen pKₛ-Wert (bzw. einen pKₛ₁-Wert) bei 25 °C von 2,5 oder weniger aufweist. Geeignete Säuren werden ausgewählt aus Phosphorsäure, Salpetersäure, Schwefelsäure, Salzsäure und Perchlorsäure, wobei Phosphorsäure, Schwefelsäure und ihre Mischungen bevorzugt sind und Schwefelsäure besonders bevorzugt ist. Ist der pH-Wert eingestellt, kann das Katalysatorsystem dem Reaktionsgemisch wieder zugeführt werden.

Bevor das Reaktionsgemisch im Falle der Membranfiltration der Membran zugeführt wird, erfolgt, sofern zwei flüssige Phasen vorhanden sind, vorzugsweise ihre Trennung. Diese kann beispielsweise mittels Phasentrennbehälter durchgeführt werden. Insofern wird das Reaktionsgemisch nicht als zweiphasiges, sondern als einphasiges Gemisch auf die Membran gegeben. Vorzugsweise wird die nicht-wässrige (organische) Phase per Membran getrennt. Der Katalysator kann auch aus der wässrige Phase mittels Membrantechnik abtrennt werden. Mithin werden wässrige und organische Phase getrennt voneinander mit einer jeweiligen Membran von Katalysator bereinigt. Für die wässrige Phase kann eine andere Membran verwendet werden als für die organische Phase. Das Retentat enthaltend die katalysatorhaltige organische Phase wird im Anschluss der Reaktivierung zugeführt.

Für die wässrige Phase wird vorzugsweise eines der folgenden Membranmaterialen verwendet: Polyamide, aromatische Polyamide, Polysulfone, Polyethersulfone, hydrophobierte Polyethersulfone, sulfonierte Polyethersulfone, Celluloseactat, Polypiperazin und Polyvinylidenfluorid. Für die Auftrennung der organischen Phase sollte hingegen vorzugsweise eine Membran auf Basis von Siliconacrylat und/oder Polydimethylsiloxan (PDMS) und/oder Polyimid verwendet werden. Das aus beiden Phasen aufkonzentrierte Katalysatorsystem kann vereinigt werden.

Sofern keine Phasentrennung des Reaktionsgemischs eintreten sollte, sind dem Fachmann Maßnahmen zur Phasentrennung wie Erhöhung der Polarität der wässrigen Phase oder Änderung der Dichte einer Phase bekannt.

Mit dem Membranverfahren wird das Katalysatorsystem im Retentat angereichert. Unter "Retentat" versteht der Membrantechniker den Abfluss von der Membran, der vor der Membran abgezogen wird. Das Material, welches die Membran überwindet, wird "Permeat" genannt und hinter der Membran abgezogen. Die Menge an zurückgehaltenem Katalysator kann dabei anhand der Menge an zurückgehaltenem Übergangsmetall nachgewiesen werden. Das Verfahren erlaubt es insbesondere, mehr als 50 % des Übergangsmetalls, bezogen auf die Gesamtmenge an Übergangsmetall im Reaktionsgemisch vor der Filtration, im Retentat zurückzuhalten. Bevorzugt werden mehr als 70 % des Übergangsmetalls zurückgehalten, besonders bevorzugt mehr als 90 %. Falls ein Phasentransferreagenz eingesetzt wird, wird dieses in der Regel einen anderen Rückhalt erzielen als das Übergangsmetall. Dennoch wird es im Retentat angereichert. Deswegen wird das gesamte Katalysatorsystem zumindest teilweise im Retentat angereichert. Das Übergangsmetall kann durch ICP-MS (Massenspektroskopie mit induktiv gekoppeltem Plasma) oder RFA (Röntgenfluoreszenzanalyse) nachgewiesen werden.

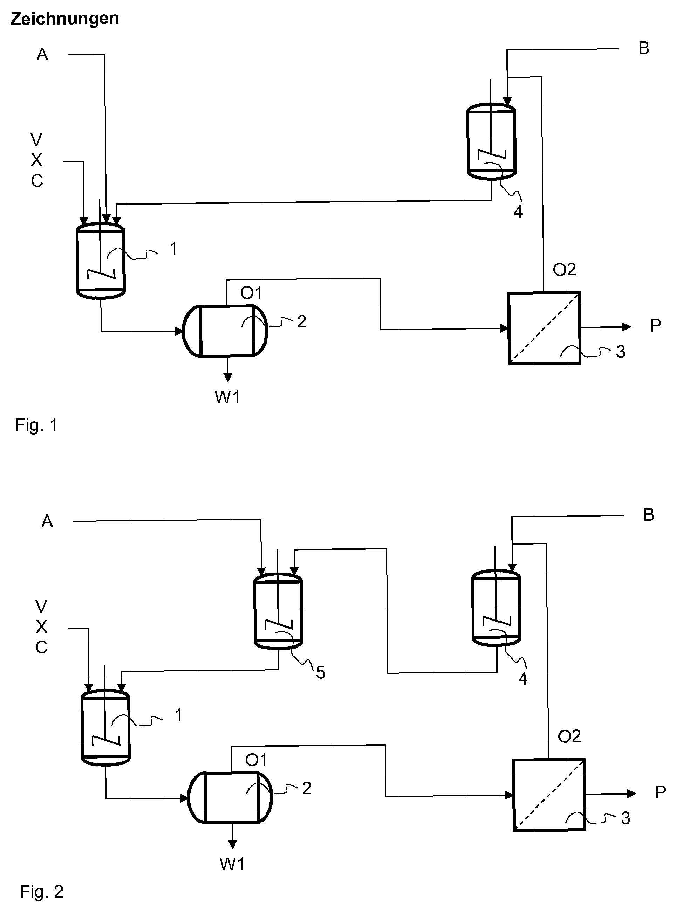

Die Figuren 1 bis 4 veranschaulichen den Prozessverlauf einer kontinuierlichen Verfahrensweise mittels Membranfiltration. Diese Verfahrensführungen stellen besonders bevorzugte Ausführungsformen der Erfindung dar. Die organische Verbindung V, vorzugsweise eine ungesättigte cyclische C6- bis C12-Verbindung, insbesondere CDEN, das Peroxid X und das Katalysatorsystem C in Form einer wässrigen Lösung werden zusammen mit einem Phasentransferreagenz in einen Rührkessel (1) gegeben. Der pH-Wert wird mittels Säure - soweit erforderlich - auf einen bevorzugten Wert ≤ 4 eingestellt. Rührkessel-Kaskaden sind möglich. Der oder die Rührkessel können auf Temperaturen von 20 °C bis 150 °C temperiert werden, wobei die Substanzen in flüssigem Zustand bleiben. Im Phasentrennbehälter (2) wird die wässrige Phase W1 abgetrennt, bevor die organische Phase 01 auf die Membran (3) trifft. Das oxidierte Produkt P kann als Permeat abgetrennt werden. Das Katalysatorsystem C wird in der organischen Phase 02 in einen weiteren Rührkessel (4) geleitet, in den eine wässrige Base B, vorzugsweise Natronlauge, gegeben wird. Die einzelnen Phasen enthalten üblicherweise:
W1: Wasser, Peroxid, Katalysatorsystem, Phasentransferreagenz
01: organische Verbindung, Produkt, ggf. organisches Lösemittel, Katalysatorsystem, Phasentransferreagenz
02: wie 01, jedoch höhere Konzentration an Katalysatorsystem.

In einer ersten bevorzugten Variante (Fig. 1) wird die Mischung aus 02 und B wieder dem Rührkessel (1) zugeführt. Bei Bedarf wird dem Rührkessel (1) Säure A zur Einstellung des pH-Werts zugeführt.

In einer zweiten bevorzugten Variante (Fig. 2) wird die Mischung aus 02 und B in einen weiteren Rührkessel (5) geleitet. Dort wird die Mischung mit einer anorganischen Säure A auf den gewünschten pH-Wert eingestellt (bevorzugt 1,5 bis 4) und wieder dem Rührkessel (1) zugeführt.

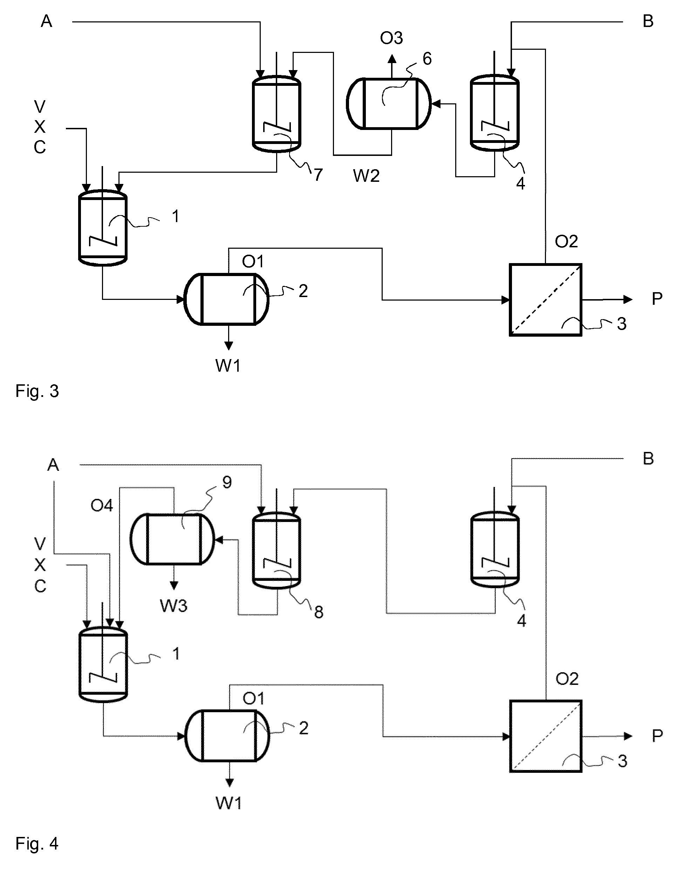

In einer dritten bevorzugten Variante (Fig. 3) wird die Mischung aus 02 und B in einen Phasentrennbehälter (6) gegeben, mit dem die wässrige Phase W2 von der organischen Phase 03 abgetrennt wird. W2 wird in einen Rührkessel (7) geführt, in dem weiterhin die Säure A eingebracht und auf den gewünschten pH-Wert eingestellt (bevorzugt 1,5 bis 4) wird. Die Mischung aus W2 und A wird dem Rührkessel (1) im Kreislauf wieder zugeführt.
W2: Wasser, Base, Katalysatorsystem
03: wie 02, jedoch ohne Katalysatorsystem

Alternativ kann die dritte Variante ohne Membran (3) durchgeführt werden. Hierbei wird 01 direkt in den Rührkessel (4) überführt. Das Produkt verbleibt in der organischen Phase und kann als 03 abgetrennt werden.

Fig. 4 demonstriert eine vierte bevorzugte Variante, in der die Mischung aus 02 und B in einen Rührkessel (8) überführt und mit der Säure A vermischt wird. Mit der Säure wird auf den gewünschten pH-Wert eingestellt (bevorzugt 1,5 bis 4). Die resultierende Mischung aus 02, B und A gelangt in einen Phasentrennbehälter (9), in dem die wässrige Phase W3 abgetrennt und die verbleibende organische Phase 04 dem Rührkessel (1) im Kreislauf wieder zugeführt wird. Auf Grund der Abtrennung der wässrigen Phase W3 kann weiterhin Säure A in den Rührkessel 1 geleitet werden.
W4: Wasser, Salz der anorganischen Säure und der Base
04: wie 02, jedoch ohne Katalysatorsystem

Die genannten Phasen können weitere Bestandteile enthalten; insbesondere erfolgen Abtrennungen in der Regel nicht quantitativ. Die anorganische Säure A wird vorzugsweise ausgewählt aus Phosphorsäure, Schwefelsäure und Mischungen daraus, wobei Schwefelsäure besonders bevorzugt ist.

Mit dem erfindungsgemäßen Verfahren ist es möglich, homogene Katalysatoren nach Katalyse von Oxidationsreaktionen zurückzugewinnen. Dies ermöglicht eine besonders wirtschaftliche Reaktionsführung, da der Katalysator zurückgewonnen und reaktiviert werden kann. Eine Zugabe an frischem Katalysator ist nur noch im geringen Maß erforderlich.

Ein weiterer Gegenstand der Erfindung ist ein Verfahren zur Oxidation organischer Verbindungen. Hierbei wird ein homogenes Oxidations-Katalysatorsystem eingesetzt, das nach dem zuvor geschriebenen Verfahren reaktiviert worden ist.

Ein weiterer Gegenstand der Erfindung ist ein Verfahren zur Synthese von Lactamen, vorzugsweise Laurinlactam (erfindungsgemäßes Lactamverfahren). In einer ersten Stufe wird die organische Verbindung in Form einer cyclischen, ungesättigten Verbindung, vorzugsweise C6- bis C12-Verbindung, bevorzugt C12-Verbindung, besonders bevorzugt CDEN, zum Epoxid oxidiert. Die Oxidation erfolgt in Gegenwart des homogenen Oxidations-Katalysatorsystems, das nach dem oben genannten Verfahren reaktiviert wird.

Die epoxidierten Verbindungen können im Anschluss in Anwesenheit von einem Katalysator umfassend ein Edelmetall und ein Metalloxid zum entsprechenden Keton umgesetzt werden. Während der Umlagerung oder im Anschluss dessen kann Wasserstoff zugegeben werden, womit das Alkohol-Derivat entstehen kann. Sofern das Keton in einem Gemisch mit dem Alkohol-Derivat vorliegt, kann eine Dehydrierung des Alkohols zum Keton erfolgen. Anschließend kann das Keton oximiert werden. Im Folgeschritt kann die Beckmann-Umlagerung zum Lactam erfolgen, wobei die Umlagerung mittels Schwefelsäure oder Cyanurchlorid durchgeführt kann. Die Lactame können unter Polykondensation zu Polyamiden weiterverarbeitet werden.

Die Umlagerung, die Dehydrierung, die Oximierung, die Beckmann-Umlagerung sowie die Kondensationsreaktion sind dem Fachmann bekannt.

In einer bevorzugten Ausführungsform des erfindungsgemäßen Lactamverfahrens wird aus CDANepoxid Laurinlactam hergestellt, das unter Polymerisation zu Polyamid 12 reagiert.

Im Rahmen des bevorzugten Lactamverfahrens CDEN gewonnen werden durch folgende Reaktionsschritte: 1,3-Butadien wird durch Cyclotrimerisation zu Cyclododecatrien umgesetzt. Anschließend erfolgt eine Hydrierung zum Cyclododecen.

### Beispiel 1 (nicht erfindungsgemäß)

### Kontinuierliche Verfahrensweise ohne Reaktivierung

Es wurde eine Epoxidierung cyclischer, ungesättigter C12-Verbindungen in einer 3-stufigen Rührkesselkaskade kontinuierlich durchgeführt. Die verwendete Rührkessel-Kaskade beinhaltete 2 Reaktoren mit einem Nennvolumen von je 5 Litern und als letzte Stufe einen Rührkessel mit einem Nennvolumen von 25 Litern. Die drei Reaktoren waren mit einem Mantel versehen und wurden über diesen auf eine Temperatur von 80 °C temperiert.

1,5 kg/h einer cyclischen, ungesättigten C12-Verbindung (81 Gew.-% CDEN und 19,1 Gew.-% CDAN), Alamin (Trioctylamin als Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 1,08 mol H₂O₂ pro mol CDEN zugegeben.

Das aus zwei flüssigen Phasen bestehende Reaktionsgemisch gelangte von der Kaskade in einen Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. einer nominalen Membranfläche von 0,6 m² eingesetzt. Die aktive Trennschicht der Membranen besteht aus Siliconacrylat und die Trägerschicht besteht aus Polyimid.

79% des Feeds zur Membrananlage wurde als Permeat gewonnen. Das Retentat (21% des Feeds zur Membranlage), welches das Katalysatorsystem enthält, wurde zurück in die Reaktion geführt.

Die wässrige Phase aus dem Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembranen Desal DK von GE Power & Water mit einer nominalen Membranfläche von 0,7 m² eingesetzt. 70% des Feeds zur Membrananlage wurde als Permeat gewonnen. Das Retentat (30% des Feeds zur Membranlage), welches das Katalysatorsystem enthält, wurde zurück in den ersten Reaktor der Kaskade geführt.

Zusätzlich zu der Rückführung des Katalysatorsystems mit den Retentaten aus den Membrananlagen wurden Alamin, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Alamin |
|---|---|---|---|
| Menge bezogen auf zugeführte CDEN-Menge | 0,00239 g/g | 0,029 g/g | 0,0046 g/g |

Mit diesen Katalysator-Kennzahlen ergab sich ein Gesamtumsatz für den CDEN-Anteil von 94,5%.

Nach einer Betriebszeit der Versuchsanlage von 240 Stunden wurde eine Konzentration an Wolfram im ersten Reaktor der Kaskade von 1,08 mol-% Wolfram bezogen auf CDEN im Zulauf über ICP-Massenspektrometrie ermittelt. Zusätzlich wurde Feststoffablagerungen in der Versuchsanlage beobachtet, die dazu führten, dass die Verbindungsleitungen zwischen Phasentrennbehälter und Membrananlage verstopften und ein weiterer Betrieb der Versuchsanlage nicht mehr möglich war.

### Beispiel 2 (erfindungsgemäß)

### Kontinuierliches Verfahren mit Reaktivierung

Im erfindungsgemäßen Beispiel wurde der Versuchsaufbau aus Beispiele 1 um eine Laugenbehandlung für das Retentat und eine anschließenden Säurezugabe ergänzt (analog Fig. 2).

2,3 kg/h einer cyclischen, ungesättigten C12-Verbindung (91 Gew.-% CDEN und 9 Gew.-% CDAN), Alamin (Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 0,995 mol H₂O₂ pro mol CDEN zugegeben.

Das aus zwei flüssigen Phasen bestehende Reaktionsgemisch gelangte von der Kaskade in einen Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. einer nominalen Membranfläche von 0,6 m² eingesetzt. Die aktive Trennschicht der Membranen besteht aus Siliconacrylat und die Trägerschicht besteht aus Polyimid.

79% des Feeds zur Membrananlage wurde als Permeat gewonnen. Das Retentat (21% des Feeds zur Membranlage), welches das Katalysatorsystem enthält, wurde in einen weiteren Rührkessel geführt, in den eine 0,5 M Natronlauge zugegeben wurde. Die Mischung wurde auf einen pH-Wert von 11 eingestellt. Anschließend wurde die Mischung in einen weiteren Rührkessel geleitet und dort mit Schwefelsäure versetzt und auf einen pH von 2 eingestellt. Die resultierende Mischung wurde wieder dem ersten Reaktor der Kaskade zugeführt.

Die wässrige Phase aus dem Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembranen Desal DK von GE Power & Water mit einer nominalen Membranfläche von 0,7 m² eingesetzt. 70% des Feeds zur Membrananlage wurde als Permeat gewonnen. Das Retentat (30% des Feeds zur Membranlage), welches das Katalysatorsystem enthält, wurde zurück in den ersten Reaktor der Kaskade geführt.

Zusätzlich zu der Rückführung des Katalysatorsystems wurden Alamin, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Alamin |
|---|---|---|---|
| Menge bezogen auf zugeführte CDEN-Menge | 0,0012 g/g | 0,0020 g/g | 0,006 g/g |

Mit diesen Katalysator-Kennzahlen ergab sich ein Gesamtumsatz für den CDEN-Anteil von 95%.

Nach einer Betriebszeit der Versuchsanlage von 840 Stunden wurde eine Konzentration an Wolfram im ersten Reaktor der Kaskade von 0,51 mol-% Wolfram bezogen auf CDEN im Zulauf über ICP Massenspektrometrie ermittelt. Es wurde keine Feststoffablagerungen in der Versuchsanlage beobachtet, die zu einer Abstellung der Versuchsanlage zwingen würden.

### Beispiel 3 (erfindungsgemäß)

### Kontinuierliches Verfahren mit Reaktivierung

Im erfindungsgemäßen Beispiel wurde der Versuchsaufbau aus Beispiele 2 um eine Phasentrennung nach der Laugenbehandlung für das Retentat und einer anschließenden Säurezugabe ergänzt (analog Fig. 4). Die organische Phase aus der Phasentrennung wurde in die Reaktorkaskade zurückgeführt. Die wässrige Phase wurde aus dem Prozess ausgeschleust.

2,3 kg/h einer cyclischen, ungesättigten C12-Verbindung (91 Gew.-% CDEN und 9 Gew.-% CDAN), Alamin (Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 0,979 mol H₂O₂ pro mol CDEN zugegeben.

Das aus zwei flüssigen Phasen bestehende Reaktionsgemisch gelangte von der Kaskade in einen Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. einer nominalen Membranfläche von 0,6 m² eingesetzt. Die aktive Trennschicht der Membranen besteht aus Siliconacrylat und die Trägerschicht besteht aus Polyimid.

84% des Feeds zur Membrananlage wurde als Permeat gewonnen. Das Retentat (16% des Feeds zur Membranlage), welches das Katalysatorsystem enthält, wurde in einen weiteren Rührkessel geführt, in den eine 0,5 M Natronlauge zugegeben wurde. Die Mischung wurde auf einen pH-Wert von 11 eingestellt. Anschließend wurde die Mischung in einen weiteren Rührkessel geleitet und dort mit Schwefelsäure versetzt und auf einen pH von 2 eingestellt. Anschließend wurde die Mischung einem Phasentrennbehälter zugeführt. Die wässrige Phase wurde aus dem Prozess ausgeschleust. Die resultierende organische Phase wurde wieder dem ersten Reaktor der Kaskade zugeführt.

Die wässrige Phase aus dem Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembranen Desal DK von GE Power & Water mit einer nominalen Membranfläche von 0,7 m² eingesetzt. 83% des Feeds zur Membrananlage wurde als Permeat gewonnen. Das Retentat (17% des Feeds zur Membranlage), welches das Katalysatorsystem enthält, wurde zurück in den ersten Reaktor der Kaskadegeführt.

Zusätzlich zu der Rückführung des Katalysatorsystems wurden Alamin, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Alamin |
|---|---|---|---|
| Menge bezogen auf zugeführte CDEN-Menge | 0,0012 g/g | 0,0035 g/g | 0,004 g/g |

Mit diesen Katalysator-Kennzahlen ergab sich ein Gesamtumsatz für den CDEN-Anteil von 91%.

Nach einer Betriebszeit der Versuchsanlage von 500 Stunden wurde eine Konzentration an Wolfram im ersten Reaktor der Kaskade von 0,42 mol-% Wolfram bezogen auf CDEN im Zulauf über ICP Massenspektrometrie ermittelt. Es wurde keine Feststoffablagerungen in der Versuchsanlage beobachtet, die zu einer Abstellung der Versuchsanlage zwingen würden.

### Beispiel 4 (erfindungsgemäß)

### Kontinuierliches Verfahren mit Reaktivierung

Im erfindungsgemäßen Beispiel wurde der Versuchsaufbau aus Beispiele 2 verwendet.

2,0 kg/h einer cyclischen, ungesättigten C12-Verbindung (91 Gew.-% CDEN und 9 Gew.-% CDAN), Trioctylammoniummethyl-sulfat (Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 1 mol H₂O₂ pro mol CDEN zugegeben.

Das aus zwei flüssigen Phasen bestehende Reaktionsgemisch gelangte von der Kaskade in einen Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. einer nominalen Membranfläche von 0,6 m² eingesetzt. Die aktive Trennschicht der Membranen besteht aus Siliconacrylat und die Trägerschicht besteht aus Polyimid.

84% des Feeds zur Membrananlage wurde als Permeat gewonnen. Das Retentat (16% des Feeds zur Membranlage), welches das Katalysatorsystem enthält, wurde in einen weiteren Rührkessel geführt, in den eine 0,5 M Natronlauge zugegeben wurde. Die Mischung wurde auf einen pH-Wert von 11 eingestellt. Anschließend wurde die Mischung in einen weiteren Rührkessel geleitet und dort mit Schwefelsäure versetzt und auf einen pH von 2 eingestellt. Die resultierende Mischung wurde wieder dem ersten Reaktor der Kaskade zugeführt.

Die wässrige Phase aus dem Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembranen Desal DK von GE Power & Water mit einer nominalen Membranfläche von 0,7 m² eingesetzt. 55% des Feeds zur Membrananlage wurde als Permeat gewonnen. Das Retentat (45% des Feeds zur Membranlage), welches das Katalysatorsystem enthält, wurde zurück in den ersten Reaktor der Kaskadegeführt.

Zusätzlich zu der Rückführung des Katalysatorsystems wurden Trioctylammoniummethyl-sulfat, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Trioctylammoniummethyl-sulfat |
|---|---|---|---|
| Menge bezogen auf zugeführte CDEN-Menge | 0,0006 g/g | 0,0035 g/g | 0,0016 g/g |

Mit diesen Katalysator-Kennzahlen ergab sich ein Gesamtumsatz für den CDEN-Anteil von 95%.

Nach einer Betriebszeit der Versuchsanlage von 790 Stunden wurde eine Konzentration an Wolfram im ersten Reaktor der Kaskade von 0,52 mol-% Wolfram bezogen auf CDEN im Zulauf über ICP Massenspektrometrie ermittelt. Es wurde keine Feststoffablagerungen in der Versuchsanlage beobachtet, die zu einer Abstellung der Versuchsanlage zwingen würden.

## Patentansprüche

1. Verfahren zur Reaktivierung eines Katalysatorsystems, wobei
• eine organische Verbindung in einem Reaktionsgemisch oxidiert wird,
• das Reaktionsgemisch ein homogenes Katalysatorsystem zur Oxidation der organischen Verbindung und mindestens ein Peroxid umfasst, und
• das Katalysatorsystem mindestens ein Metall in seiner höchsten Oxidationsstufe umfasst, wobei das Metall ausgewählt ist aus mindestens einem Metall der Gruppen IVb, Vb und Vlb,
**dadurch gekennzeichnet, dass** die Reaktivierung des Katalysatorsystems durch Zugabe mindestens einer wässrigen Base auf einen pH-Wert ≥ 4, vorzugsweise ≥ 4,5, bevorzugt ≥ 7, besonders bevorzugt ≥ 11, erfolgt.

2. Verfahren nach Anspruch 1, **dadurch gekennzeichnet, dass** das Reaktionsgemisch eine wässrige Phase und eine organische Phase sowie einen Phasentransferreagenz umfasst.

3. Verfahren nach Anspruch 2, **dadurch gekennzeichnet, dass** die Oxidation bei einem pH-Wert ≤ 4, vorzugsweise 1,5 bis 4, erfolgt.

4. Verfahren nach Anspruch 3, **dadurch gekennzeichnet, dass** zur Einstellung des pH-Werts ≤ 4 mindestens eine anorganische Säure mit einem pKₛ-Wert (25 °C) von 2,5 oder kleiner, vorzugsweise Phosphorsäure, Schwefelsäure oder ihre Mischungen, besonders bevorzugt Schwefelsäure ausgewählt wird.

5. Verfahren nach einem der vorherigen Ansprüche, **dadurch gekennzeichnet, dass** das Katalysatorsystem vor der Reaktivierung von der oxidierten organischen Verbindung abgetrennt wird.

6. Verfahren nach Anspruch 5, **dadurch gekennzeichnet, dass** das Katalysatorsystem nach der Reaktivierung wieder dem Reaktionsgemisch zugeführt wird.

7. Verfahren nach Anspruch 6, **dadurch gekennzeichnet, dass** der pH-Wert des Katalysatorsystems nach der Reaktivierung und vor der Zuführung auf einen Wert von < 7, vorzugsweise 1,5 bis 4, eingestellt wird.

8. Verfahren nach Anspruch 6 oder 7, **dadurch gekennzeichnet, dass** eine ggf. vorhandene organische Phase nach der Reaktivierung und vor der Zuführung zum Reaktionsgemisch abgetrennt wird.

9. Verfahren nach einem der vorherigen Ansprüche, **dadurch gekennzeichnet, dass** als Peroxid Wasserstoffperoxid eingesetzt wird.

10. Verfahren nach einem der vorherigen Ansprüche, **dadurch gekennzeichnet, dass** die Base ausgewählt ist aus Ammoniak, Alkalihydroxiden oder Mischungen daraus.

11. Verfahren nach Anspruch 10, **dadurch gekennzeichnet, dass** das Alkalimetall aus Natrium und Kalium, vorzugsweise Natrium, ausgewählt ist.

12. Verfahren nach einem der vorherigen Ansprüche, **dadurch gekennzeichnet, dass** die organische Verbindung eine ungesättigte, cyclische Verbindung mit sechs bis zwölf Kohlenstoffatomen, vorzugsweise Cyclododecen, ist.

13. Verfahren nach einem der vorherigen Ansprüche, **dadurch gekennzeichnet, dass** die Oxidation ohne organisches Lösemittel erfolgt.

14. Verfahren zur Oxidation von organischen Verbindungen, **dadurch gekennzeichnet, dass** ein homogenes Oxidations-Katalysatorsystem eingesetzt wird, das nach einem der Ansprüche 1 bis 13 reaktiviert wird.

15. Verfahren zur Synthese von Lactamen, umfassend die Schritte
• Oxidation einer cyclischen, ungesättigten Verbindung zum Epoxid,
• Umlagerung zu Keton,
• Oximierung zum Oxim und
• Umlagerung zum Lactam,
wobei die Oxidation der cyclischen, ungesättigten Verbindung in Gegenwart eines homogenen Oxidations-Katalysatorsystems erfolgt, **dadurch gekennzeichnet, dass** eine Reaktivierung des Katalysatorsystems nach einem Verfahren der Ansprüche 1 bis 13 erfolgt.
